Europäisches Patentamt

European Patent Office

Office européen des brevets

(11) Veröffentlichungsnummer: **0 388 695**
**A1**

## (12) EUROPÄISCHE PATENTANMELDUNG

(21) Anmeldenummer: 90104166.5

(22) Anmeldetag: 03.03.90

(51) Int. Cl.⁵: **A61K 31/195, A61K 31/35,**
**//(A61K31/195,31:015)**

(30) Priorität: **18.03.89 DE 3909049**

(43) Veröffentlichungstag der Anmeldung:
**26.09.90 Patentblatt 90/39**

(84) Benannte Vertragsstaaten:
**BE DE DK ES FR GB GR IT LU NL**

(71) Anmelder: **Boskamp, Arthur**
**Schillerstrasse 12**
**D-2210 Itzehoe(DE)**

(72) Erfinder: **Boskamp, Arthur**
**Schillerstrasse 12**
**D-2210 Itzehoe(DE)**

(74) Vertreter: **UEXKÜLL & STOLBERG**
**Patentanwälte**
**Beselerstrasse 4**
**D-2000 Hamburg 52(DE)**

(54) **Präparat und dessen Verwendung.**

(57) Beschrieben wird ein Kombinationspräparat, das als Wirkstoffe Acetylcystein und ein etherisches Öl mit sekretolytischer und/oder sekretomotorischer Wirkung, vorzugsweise Cineol, enthält. Die erfindungsgemäße Wirkstoffkombination eignet sich insbesondere zur Herstellung von Mucolytika und Sekretolytika. Die bekannte Wirkung von Acetylcystein wird durch das etherische Öl erheblich verbessert, was sich insbesondere bei der Behandlung von Bronchitis und Sinusitis positiv auswirkt.

EP 0 388 695 A1

## Präparat und dessen Verwendung

Die Erfindung betrifft ein Arzneimittel, das eine Acetylcystein umfassende Wirkstoffkombination enthält, sowie die Verwendung der Acetylcystein umfassenden Wirkstoffkombination zur Herstellung eines Arzneimittels, insbesondere eines Mucolytikums und Sekretolytikums für die Behandlung von akuten und chronischen Erkrankungen der Luftwege.

Der Wirkstoff Acetylcystein (vgl. Merck Index) wird zur Verringerung der Viskosität des Schleimes verwendet, so daß dieser dünnflüssiger wird und leichter aus den Bronchien oder aus den Nebenhöhlen entfernt werden kann. Dementsprechend wird Acetylcystein in Antitussiva und Expektorantia verwendet (siehe Rote Liste). Ein bekanntes Acetylcystein enthaltendes Mucolytikum für die Behandlung von Nebenhöhlenentzündungen ist beispielsweise FLUIMUCIL (eingetragenes Warenzeichen).

Es wurde nun überraschend gefunden, daß die Wirksamkeit von Acetylcystein enthaltenden Präparaten durch Zusatz von etherischen Ölen, die sekretolytisch und/oder sekretomotorisch wirken, erheblich verbessert werden kann. Erfindungsgemäß geeignete etherische Öle mit sekretolytischer und sekretomotorischer Wirkung sind beispielsweise Eukalyptusöl, Limonen, α-Pinen, Terpentinöl und Cineol (siehe Merck Index). Letzteres wird ebenfalls in Antitussiva und Expektorantia verwendet (siehe Rote Liste). Ein bekanntes Cineol enthaltendes Präparat ist beispielsweise Soledum (eingetragenes Warenzeichen).

Gegenstand der Erfindung ist dementsprechend ein Arzneimittel, das Acetylcystein als Wirkstoff sowie gegebenenfalls übliche Hilfs- und Trägerstoffe enthält, das dadurch gekennzeichnet ist, daß es als weiterer Wirkstoff ein etherisches Öl mit sekretolytischer und/oder sekretomotorischer Wirkung enthält. Erfindungsgemäß bevorzugt ist ein derartiges Arzneimittel, das als etherisches Öl Cineol enthält.

Die Herstellung des erfindungsgemäßen Arzneimittels bietet keine Besonderheiten und liegt im Bereich des Könnens des Durchschnittsfachmanns. Geeignete Applikationsformen sind zum Beispiel Kapseln, Dragees, Pillen und Tabletten.

Für die Herstellung von Kapseln eignen sich herkömmliche Weichgelatinekapseln. Geeignete Gelatinemassen bestehen beispielsweise zu 30 bis 60 Gew.% aus Gelatine, zu 10 bis 40 Gew.% aus Weichmacher (z.B. Glyerin, Sorbid) und zu 15 bis 40 Gew.% aus Wasser. Es können weitere Stoffe zugesetzt werden, wie z.B. Konservierungsmittel und Farbstoffe. Die Größe der Kapsel variiert je nach Inhalt.

Die beiden Wirkstoffe bilden in der entsprechenden Dosierung gelöst in einem flüssigen organischen Trägermaterial den Kapselinhalt. Als Trägermaterial sind beispielsweise Mineralöl, pflanzliche Öle, Triglyceride, Glykole und Polyole geeignet.

Die Herstellung der Weichgelatinekapseln erfolgt in üblicher Weise mit Hilfe einer Verkapselungsmaschine. Diese wird aus zwei Flüssigkeitsbehältern beschickt, von denen einer die geschmolzene Gelatinemasse und der andere das erwärmte flüssige Füllgut enthält. Die flüssige Gelatinemasse und der Füllstoff werden zusammengefügt und zu Gelatinekapseln geformt (bezüglich weiterer Einzelheiten zur Herstellung von Weichgelatinekapseln vgl. z.B. P.H. List, Arzneiformenlehre, 4. Auflage, 1985, Seiten 345 bis 353).

Das Gewichtsverhältnis von Acetylcystein zu etherischem Öl in dem erfindungsgemäßen Arzneimittel beträgt vorzugsweise 1:100 bis 100:1 und insbesondere 1:5 bis 5:1. Eine Dosiseinheit des erfindungsgemäßen Arzneimittels enthält vorzugsweise 10 bis 1000 mg und insbesondere 60 bis 600 mg Acetylcystein und vorzugsweise 10 bis 1000 mg, insbesondere 50 bis 800 mg etherisches Öl.

Es hat sich herausgestellt, daß die Wirkstoffkombination aus Acetylcystein und etherischem Öl zu einer verbesserten Sekretentfernung bei der Behandlung von akuten und chronischen Erkrankungen der Luftwege führt. Deshalb eignet sich das erfindungsgemäße Arzneimittel insbesondere zur Behandlung von Bronchitis oder Sinusitis.

## Ansprüche

1. Arzneimittel, das Acetylcystein als Wirkstoff sowie gegebenenfalls übliche Hilfs- und Trägerstoffe enthält, **dadurch gekennzeichnet**, daß es als weiterer Wirkstoff ein etherisches Öl mit sekretolytischer und/oder sekretomotorischer Wirkung enthält.

2. Arzneimittel nach Anspruch 1, **dadurch gekennzeichnet**, daß es als etherisches Öl Cineol enthält.

3. Arzneimittel nach Anspruch 1 oder 2, **dadurch gekennzeichnet**, daß es Acetylcystein und etherisches Öl in einem Gewichtsverhältnis von 1:100 bis 100:1 und vorzugsweise 1:5 bis 5:1 enthält.

4. Arzneimittel nach einem der Ansprüche 1 bis 3, **dadurch gekennzeichnet**, daß eine Dosiseinheit 10 bis 1000 mg und vorzugsweise 60 bis 600 mg Acetylcystein und 10 bis 1000 mg und vorzugsweise 50 bis 800 mg etherisches Öl enthält.

5. Verwendung der Wirkstoffkombination aus Acetylcystein und etherischem Öl mit sekretolytischer und/oder sekretomotorischer Wirkung zur Herstellung eines Arzneimittels, insbesondere eines Mucolytikums und Sekretolytikums.

Patentansprüche für folgende Vertragsstaaten: ES, GR

1. Verfahren zur Herstellung eines Arzneimittels, dadurch gekennzeichnet, daß man Acetylcystein, ein etherisches Öl mit sekretolytischer und/oder sekretomotorischer Wirkung und ggf. übliche Hilfs- und Trägerstoffe miteinander kombiniert.

2. Verfahren nach Anspruch 1, bei dem man als etherisches Öl Cineol verwendet.

3. Verfahren nach Anspruch 1 oder 2, bei dem man Acetylcystein und etherisches Öl in einem Gewichtsverhältnis von 1:100 bis 100:1 und vorzugsweise 1:5 bis 5:1 miteinander kombiniert.

4. Verfahren nach einem der Ansprüche 1 bis 3, bei dem man Acetylcystein, etherisches Öl und ggf. übliche Hilfs- und Trägerstoffe zu Kapseln, Dragees, Pillen und Tabletten verarbeitet.

5. Verfahren nach einem der Ansprüche 1 bis 4, bei dem man so viel Acetylcystein und etherisches Öl verwendet, daß eine hergestellte Dosiseinheit 10 bis 1000 mg und vorzugsweise 60 bis 600 mg Acetylcystein und 10 bis 1000 mg und vorzugsweise 50 bis 800 mg etherisches Öl enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | "Rote Liste", 1981, Nr. 23156B, Editio Cantor, Aulendorf, DE; "Fluimucil" <br> * Fluimucil * | 1-5 | A 61 K 31/195 <br> A 61 K 31/35 // <br> (A 61 K 31/195 <br> A 61 K 31:015) |
| A,D | "Rote Liste", 1981, Nr. 23164.5B, Editio Cantor, Aulendorf, DE; "Soledum Kapseln" <br> * Soledum * | 2 | |

RECHERCHIERTE
SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 25-06-1990 | LEHERTE C.F.M. |